# EUROPEAN PATENT SPECIFICATION

(11) **EP 1 339 451 B1**
(45) Date of publication and mention of the grant of the patent: **30.09.2009**
(21) Application number: 01988608.4
(22) Date of filing: 26.10.2001
(51) Int. Cl.: A61N 1/36, A61N 1/365

(54) **APPARATUS TO MINIMIZE THE EFFECTS OF A CARDIAC INSULT**
VORRICHTUNG ZUR MINIMIERUNG DER WIRKUNGEN EINES HERZINSULTS
APPAREIL DESTINES A MINIMISER LES EFFETS D'UN ACCIDENT CARDIAQUE

(30) Priority: 26.10.2000 US 243393 P
(43) Date of publication of application: 03.09.2003
(73) Proprietor: MEDTRONIC, INC., Minneapolis, MN 55432-5604 (US)
(72) Inventor: HILL, Michael, R., S., Minneapolis, MN 55304 (US); KING, Gary, W., Fridley, MN 55432 (US); MULLEN, Thomas, J., Ham Lake, MN 55304 (US); ZHOU, Xiaohong, Plymouth, MN 55446 (US); MEHRA, Rahul, Stillwater, MN 55082 (US)
(74) Representative: Hughes, Andrea Michelle
(86) International application number: PCT/US2001/045698
(87) International publication number: WO 2002/034330

(56) References cited:
- EP-A- 0 688 577
- US-A- 5 058 584
- US-A- 5 199 428
- US-A- 5 824 021
- US-A- 6 058 331
- US-A- 6 134 470

## Description

### FIELD OF THE INVENTION

This invention relates generally to apparatus for electrically stimulating select nerves to alter conditions within the heart, and, more particularly, to nerve stimulation to protect myocardium acutely, and to reduce anginal pain by stimulating subcutaneous tissue.

### DESCRIPTION OF THE RELATED ART

Various cardiac conditions, such as supraventricular arrhythmias, angina pectoris, and ventricular dysfunction or heart failure, have been treated by electrical stimulation of the spinal cord, vagus and other nerves. Typically, electrodes are implanted in the patient adjacent the spinal area and electrically excited to produce desirable effects on the functioning of the heart. For example, a paper entitled "Vagal Tuning" by Bilgutay et al., published in the Journal of Thoracic and Cardiovascular Surgery, Vol. 56, No. 1, July 1968, pp. 71-82, discusses a system that delivers electrical stimulation to the vagus nerve using silastic coated, bipolar electrodes, such as those described in U.S. Patent No. 3,421,511. The electrodes are surgically implanted around the intact nerve or nerves and a controlled current is delivered thereto. The electrodes pass the current to the nerve(s), producing a decreased heart rate while still preserving sinus rhythm in the patient. Low amplitude stimulation has also been employed to control induced tachycardias and ectopic beats.

Angina pectoris and paroxysmal atrio-ventricular junctional or supraventricular tachycardias have also been treated by stimulating the carotid sinus nerve via implanted electrodes. For example, a paper entitled "Carotid Sinus Nerve Stimulation in the Treatment of Angina Pectoris and Supraventricular Tachycardia," published in California Medicine, 112:41-50, March 1970, describes a system in which patients may electrically stimulate their carotid sinus nerve when they sense angina and/or supraventricular tachycardia.

Delivery of electrical stimulation to the nervous system using an implanted electrode has been found particularly effective in the relief of chest pain, such as angina pectoris, that often accompanies myocardial ischemia. For example, U.S. Patent Number 5,058,584 to Bourgeois discloses a system and method for treating such chest pain using electrical stimulation within the epidural space of the spinal cord. This treatment is provided only after a symptomatic level of activity is reached as sensed by an accelerometer or other activity sensor. Similarly, U.S. Patent Number 6,058,331 to King
discusses a system and method for treating ischemia by automatically adjusting electrical stimulation to the spinal cord, peripheral nerve, or neural tissue ganglia based on a sensed patient condition. U.S. Patent Number 5,199,428 to Obel et al. discloses a system for stimulating the epidural space with
continuous and/or phasic electrical pulses using an implanted pulse generator upon the detection of myocardial ischemia to decrease cardiac workload, and thereby reduce cell death related to the ischemic event. U.S. Patent Number 5,824,021 to Rise discusses a system and method for providing spinal cord
stimulation to relieve angina, and to further provide a patient notification that an ischemic event is occurring. This spinal cord stimulation is provided only after the ischemia is already detected.

In addition to the above-described systems, other systems have been disclosed to provide nerve stimulation following the onset of predetermined condition. U.S. Patent Number 6,134,470 to Hartlaub describes a system for utilizing spinal cord stimulation to terminate tachyarrhythmias. The stimulation is provided only after the tachyarrhythmias, or a precursor thereto, has been detected. U.S. Patent Number 3,650,277 discloses a system for stimulating the left and right carotid sinus nerves in response to the detection of elevated mean arterial blood pressure to alleviate hypertension.

Each of the nerve stimulation systems described above have at least one significant drawback. For example, these nerve stimulation systems rely upon electrodes that are surgically implanted adjacent the spine, e.g., inside the vertebral canal. Successful placement of the electrodes in the region surrounding the spine requires substantial surgical expertise. Emergency personnel, however, do not commonly possess this expertise, nor do they often have the equipment or environment suitable for the task.
Thus, while emergency personnel may be summoned to transport an afflicted patient to a hospital and, thus, are the first medical personnel to administer aid to the patient, they are generally not capable of implanting electrodes. Without the implanted electrodes, the therapeutic stimulation has not heretofore been available immediately. Rather, application of the therapy is delayed until the patient arrives at an appropriate medical facility.
Furthermore, systems for chronic stimulation either have the drawback of requiring sophisticated implant techniques, or, for TENS, use electrodes that cause skin breakdown and other problems and inconvenience.

EP 0688577 A1 describes a device for treating atrial tachyarrhythmia comprising stimulation means devised to be placed in the neck area.

The present invention is directed to overcoming, or at least reducing the effects of, one or more of the problems set forth above.

### SUMMARY OF THE INVENTION

The current invention involves a neuromodulation system to provide stimulation to ar least a portion of the nervous system of the body. The invention is defined by the claims. Devices not according to the claims are disclosed for mere illustration of the invention.

The present invention provides an apparatus for protecting cardiac tissue from insult, comprising: a paddle lead coupled to first and second subcutaneous electrodes, said first electrode being provided on a first side of said lead and said second electrode being provided on a second side of said lead, said electrodes being positionable proximate to nerve tissue; and controller adapted to deliver electrical stimulation to the first and second electrodes.

The apparatus may also comprise: at least one electrode positionable proximate to one or more of the group consisting of peripheral nerves, intrinsic ' cardiac nerves, sympathetic ganglia, and cranial nerves; and a controller adapted to deliver electrical stimulation to the at least one electrode.

The apparatus may further comprise:
at least one electrode positionable subcutaneously, wherein the electrode is capable of stimulating at least a portion of a nervous system of the body; trigger means; and means for delivering electrical stimulation to the at least on electrode in response to activation of the trigger means.

The stimulation is provided using one or more subcutaneous electrodes or electrodes to stimulate e.g. peripheral nerves, intrinsic cardiac neurons, autonomic ganglia, and cranial nerves. The stimulation may be provided in anticipation or detection of a cardiac insult,
wherein "cardiac insult" in this context is intended to include, but is not limited to, angina, and mechanical, chemical, or electrical impairment or damage of cardiac tissue due to conditions such as heart failure, ventricular tachycardia, supraventricular tachycardia, ischemia, imbalance of autonomic tone, or the like.

In one embodiment, the current invention provides a system to provide stimulation at locations adjacent the spinal cord and on the chest wall. Such stimulation has been shown to improve cardiac function, to limit ischemic attacks, to reduce sympathetic activity of the cardiac tissue, and to reduce the likelihood and/or the severity of ventricular arrhythmia. Thus, the electrical stimulation produces effects similar to those induced by prescription beta-blocker drugs. This type of stimulation has been shown to reduce cardiac work, improve heart function, vasodilate peripheral arterioles and increase blood flow to the limbs.

According to a use of the invention, one or more electrodes may be placed subcutaneously adjacent one or more of the spinal vertebrae, with the T1-T4 locations being preferred, or subcutaneously near cervical nerves, with the C1-C3 location being preferred.
Alternatively, the electrodes may be placed adjacent the chest wall or anywhere within a region of the T1-T5 spinal nerves, or adjacent to peripheral nerves such as the median or ulnarnerves, or cardiac fat pods, or sympathetic ganglia, or cranial nerves. The position of the electrodes may be, for example, in the pectoral region of the left chest located beneath the facia on the muscle and motor point of the pectoral muscle with stimulation of the musculocutaneous and thoracic nerves. In another example, the electrodes may be positioned in the auxiliary region beneath the left arm with stimulation provided to the musculocutaneous, brachialcutaneous and thoracodorsal nerves. In yet another device not according to the invention one or more subcutaneous electrodes are proximate to the external housing of an implanted device to stimulation nerves adjacent to the device.

Because subcutaneous electrodes are utilized, a surgeon is not needed to position the electrodes in the patient's body. Rather, in one embodiment of the invention, a paramedic may position the one or more electrodes subcutaneously to initiate emergency treatment, for example.

According to one embodiment of the invention, the invention delivers electrical stimulation when the system is activated by a patient or other person such as a health care provider. For example, a medical care provider such as a paramedic may initiate stimulation to treat a patient that is having a heart attack. The patient himself may initiate such therapy if the onset of a heart attack is suspected. A patient may alternatively initiate stimulation in anticipation of undergoing exercise. A surgeon may initiate stimulation in anticipation of performing a surgical procedure such as the insertion of a stent, or any other procedure that may disrupt cardiac tissue. Nerve stimulation may be manually initiated by a paramedic after a high-voltage shock is delivered to a patient. Such stimulation stabilizes the heart and prevents the re-occurrence of fibrillation or an arrhythmia. Such stimulation may continue throughout the insult, and may optionally continue for a predetermined period of time following the insult.

According to another embodiment, the inventive system may be operated in a closed-loop mode. In this mode, one or more physiological parameters may be sensed using physiological sensors. The sensed physiological signals may be used to predict or detect the onset of an insult. These signals may also be used to modulate delivery of the stimulation parameters such as pulse width, amplitude, frequency, and the like.

According to yet another embodiment, the inventive system stores data signals indicative of past electrical stimulation so that future stimulation may be optimized. This stored data may also be used by healthcare professionals for treatment and diagnosis.

Preferred embodiments will now be described by way of example only, with reference to the drawings.

### BRIEF DESCRIPTION OF THE DRAWINGS

Figure 1A illustrates a stylized representation of a posterior view of a patient with electrodes positioned thereon;
Figure 1B illustrates a stylized representation of an anterior view of a patient with electrodes positioned thereon;
Figure 1C is a diagram illustrating an implantable stimulation device implanted within a patient.
Figure 2 illustrates a stylized block diagram of a controller of Figure 1;
Figure 3 illustrates a stylized control diagram of a control routine that may be performed by the controller of Figures 1 and 2;
Figure 4 illustrates a stylized flowchart of a control routine that may be performed by the controller of Figures 1 and 2;
Figure 5A is a flowchart illustrating delivery of stimulation prior to planned cardiac interventions, like bypasses, angioplasties or stents procedures;
Figure 5B is a flowchart illustrating delivery of stimulation at a particular time of day;
Figure 5C is a flowchart illustrating delivery of stimulation initiated because a patient anticipates physical activity and manually triggers therapy;
Figure 5D is a flowchart illustrating stimulation initiated at the first signs of activity in an anticipatory manner, or at the first indication that an insult may be predicted; and
Figure 5E is a flowchart illustrating stimulation initiated based on a real time recording of ischemic burden and total ischemic burden.
Figure 5F illustrates the delivery of the therapy for protection during a suspected heart attack.
Figure 6A and 6B are side views of a subcutaneous electrode according to one embodiment of the invention.
Figure 7 is a side view of an implantable medical device having a housing carrying multiple electrodes.

### DETAILED DESCRIPTION OF SPECIFIC EMBODIMENTS

Illustrative embodiments of the invention are described below. In the interest of clarity, not all features of an actual implementation are described in this specification. It will of course be appreciated that in the development of any such actual embodiment, numerous implementation-specific decisions must be made to achieve the developers' specific goals, such as compliance with system-related and business-related constraints, which will vary from one implementation to another. Moreover, it will be appreciated that such a development effort might be complex and time-consuming, but would nevertheless be a routine undertaking for those of ordinary skill in the art having the benefit of this disclosure.

Illustrative embodiments of apparatus for providing improved cardiac function according to the present invention are shown in the Figures. As will be readily apparent to those skilled in the art upon a complete reading of the present application, the present method and apparatus are applicable to a variety of systems other than the embodiment illustrated herein.

In the illustrated embodiments, apparatus for performing subcutaneous electrical stimulation to proactively modulate autonomic effects on the cardiovascular system is provided. Use of the stimulation minimizes arrhythmia, heart failure, and damage to cardiac myocytes due to the occurrence of a predicted and subsequent ischemic event. Such stimulation may be provided to one or more portions of the nervous system to also promote electrical stability of the heart and to prevent or reduce the chance for a subsequent episode involving fibrillation. As described in greater detail below, the current apparatus may employ a closed-loop control mechanism to initiate and regulate this stimulation.

Generally, the instant invention is directed to apparatus for improving the efficiency of operation of the heart and may be used to reduce the likelihood of imminent cardiac insults. Therapeutic benefits associated with the instant invention may be derived from application of the instant invention to a wide variety of cardiac conditions. Thus, as used in the instant application, the phrase "cardiac insult" is intended to include, but is not limited to angina, and damage or mechanical, chemical, or electrical impairment of cardiac tissue due to conditions such as heart failure, ventricular tachycardia, supraventricular tachycardia, ischemia, imbalance of autonomic tone, or the like. In the illustrated embodiment, the current invention may also be utilized to treat ventricular dysfunction or heart failure.

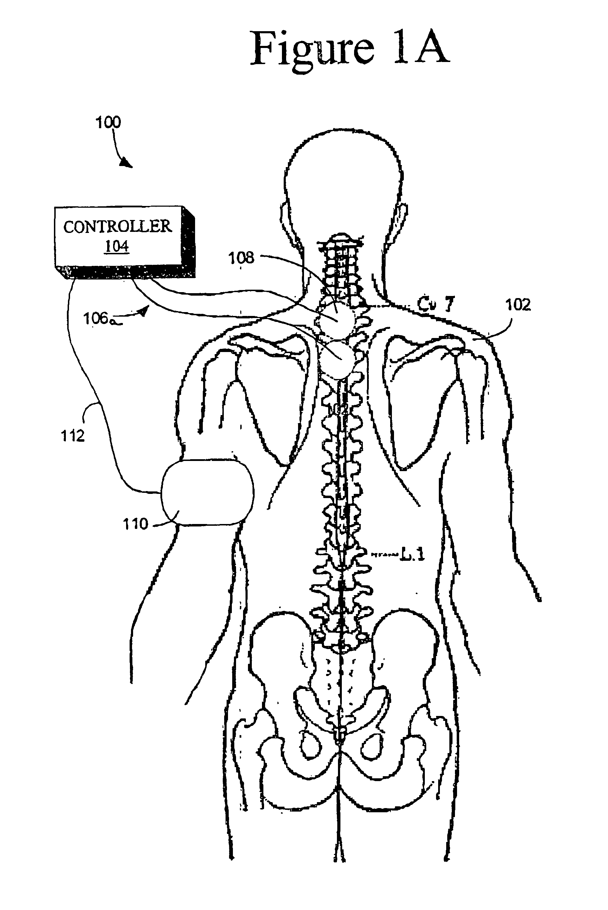

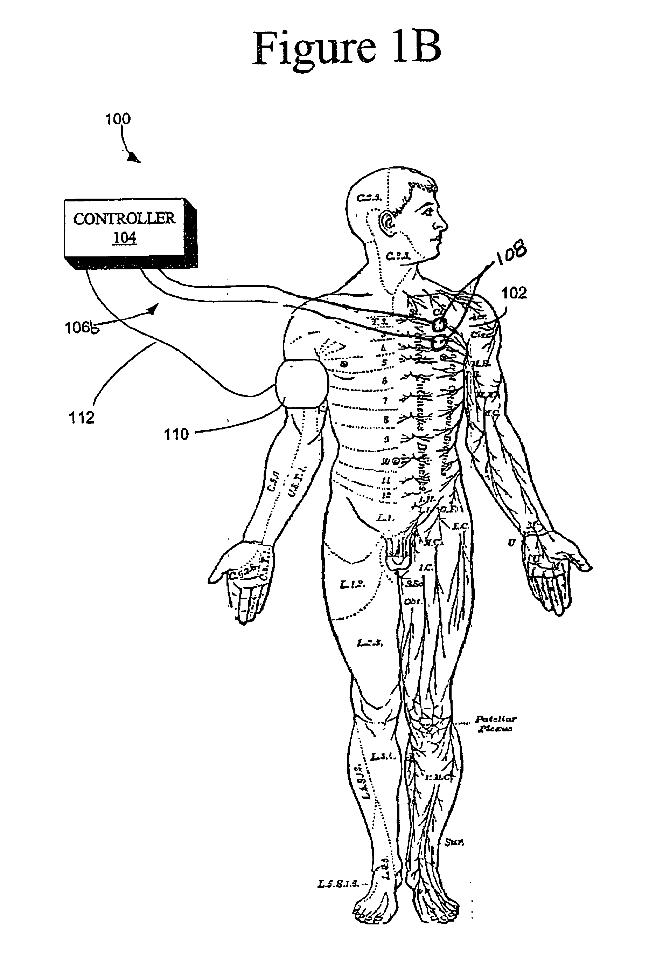

As shown in Figures 1A and 1B, an external system 100 provides stimulation to a patient 102 at locations adjacent the spinal region and on the chest wall using leads 106a and 106b, respectively. The leads are each coupled to one or more subcutaneous electrodes, as will be discussed further below. Such stimulation has been shown to improve contractibility, to further improve the pressure-volume relationship within the heart, and to reduce sympathetic activity of the cardiac tissue to reduce the likelihood of ventricular arrhythmias. Thus, the electrical stimulation produces effects similar to those induced by prescription beta-blocker drugs. The stimulation may further cause the production of neuropeptides such as CGRP, NO, and VIP that are known vasodilators, which may assist in redirection of blood flow from regions of high flow to regions of low flow. This further improves the efficiency of the heart. In ischemic dilated cardiomyopathy patients, this therapy may suppress or reduce subendocardial ischemia, and hence be cardio-protective. Electrical stimulation may further result in improvements in operational efficiency and function of cardiac tissue even in the presence of reduced blood supply.

When a subcutaneously-placed electrode is utilized, the electrodes may be placed adjacent any of the T1-T12 vertebrae or in any of the C1-C8 locations, and most preferably, any of the T1-T4 vertebrae (see Fig. 1A). Alternatively, the electrodes may be placed adjacent the chest wall (see Fig. 1B), or spinal nerves, or adjacent to peripheral nerves such as the median or ulnarnerves, or cardiac fat pods, or sympathetic ganglia, or cranial nerves. The electrodes 108 may take on any of a variety of forms of subcutaneous electrodes, as will be discussed further below. Conventional subcutaneous electrodes may be surgically inserted into the patient's body. In fact, subcutaneous stimulation may be provided using leads of the type that are commonly used for pacing the heart The implantable electrodes may be placed subcutaneously to stimulate underlying muscles, overlying cutaneous nerves, passing somatic nerves, or a combination thereof. For example, various commercially available leads, such as the Pisces®, Pisces Quad Plus ®, and Octad ® model leads, commercially-available from Medtronic Corporation, are examples of leads that may be used for this purpose. This subcutaneous placement may be desirable in emergency situations such as en route to a medical care facility following a heart attack.

As discussed above, subcutaneous electrodes may be carried on leads and inserted near nerve tissue using a delivery device such as a needle. In other instances, subcutaneous electrodes may be carried on the surface of an implanted medical device such as disclosed in commonly-assigned U.S. Patent No. 5,292,336.

Alternatively, such electrodes may be electrically-isolated from the can, as disclosed in commonly-assigned U.S. Patent No. 5,331,966.

In one embodiment, a paddle-type (flat) lead having a surface area between one square cm and five square inches or more may be used to accomplish the subcutaneous stimulation. Such a lead may be formed of an insulative material, with programmable electrodes on the flat sides of the lead for either skin stimulation, muscle stimulation, or both. According to this embodiment, the paddle-type lead may be between four and ten millimeters wide so as to be readily passable through a needle such as a twelve-gage needle before it unfolds. In one embodiment, the special delivery needle includes an oval or rectangular cross-section of appropriate size to allow for passage of the lead. Electrodes are provided on both sides of the paddle lead.

Subcutaneous electrodes are provided on both sides of the lead, the electrodes employed for stimulation at a given time may be selectively enabled by a user. Alternatively, the system may be programmable to select the type of tissue to be stimulated. This is desirable since in some vertebral instances, it may be beneficial to provide stimulation to only major nerves entering the column, whereas in other instances it may be desirable to also stimulate skin, muscle, or any combination of the nervous tissues. Various electrode combinations could be provided to allow for selective enabling of the stimulation in this manner.

One or more subcutaneous electrodes are coupled to controller 104 so that electrical signals supplied by the controller 104 provide electrical stimulation to nervous tissue in the skin, muscle, or spinal canal of the patient. The controller 104 may take the form of an external device as shown in Figures 1A and 1B. This is useful in providing therapeutic signals to a patient who is anticipating exertion or any other type of event that may cause ischemia.

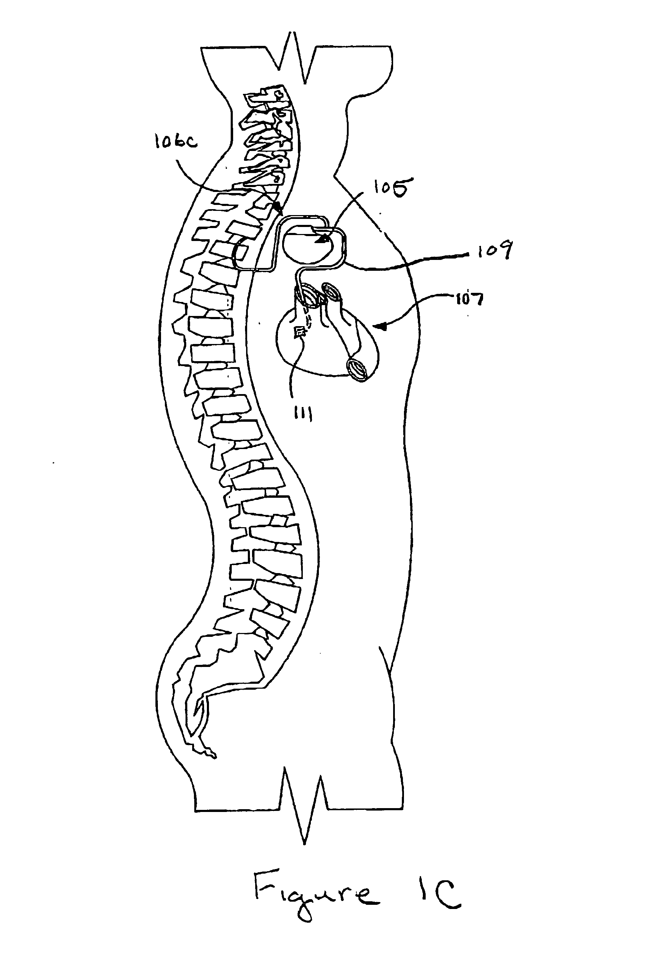

In those situations in which a patient has a history of cardiac events, it is generally useful to construct the controller 104 in a housing 105 designed to be implantable within the human body, as shown in Figure 1C. In this embodiment, implanted lead 106c is employed to deliver stimulation using a subcutaneous electrode that may be positioned under the skin using a "tunneling" approach. This housing may optionally include a pacing and/or cardioverter/defibrillator stimulation circuit for generating cardiac stimulation signals to the heart 107 using one or more leads 109, as is known in the art. Leads 109 may carry one or more physiological sensors 111 for sensing physiological signals, as is discussed below. Additionally, or in the alternative, the housing may also include a drug delivery device such as a drug pump coupled to a drug delivery catheter that may be used with the nerve stimulation to prevent anticipated physiological insults.

In one embodiment, controller 104 may be programmed for either automatic or manual operation. Manual activation of stimulation may be prompted by a variety of situations. For example, a medical care provider such as a paramedic may deliver one or more subcutaneous electrodes to an area proximate nerve tissue such as in the T1-T4 region, or in the area of referred pain, then initiate stimulation to treat a patient that is having a heart attack. A surgeon may likewise initiate this type of therapy prior to performing a surgical procedure such as the insertion of a stent, or any other procedure that may disrupt cardiac operation. Subcutaneous nerve stimulation may be manually initiated by a paramedic after a high-voltage shock is delivered to a patient. Such stimulation stabilizes the heart and prevents the re-occurrence of fibrillation or an arrhythmia. Any other anticipated or occurring cardiac insult may prompt a healthcare provider or patient to trigger controller 104 to initiated stimulation via the one or more subcutaneously-placed electrodes. Such stimulation may continue throughout the insult, and may optionally continue for a predetermined period of time following the insult. Anticipatory delivery of cardiac stimulation has been determined by the Applicants to minimize damage of cardiac myocytes due to a subsequent ischemic event. These embodiments are based on data obtained through research conducted over several years involving electrical stimulation to reduce angina.

In another instance, subcutaneous stimulation could be provided at a sub-threshold level for paresthesia during the delivery of the defibrillation shock to reduce the perceived pain associated with the arrhythmia and the shock and stabilize the heart and help prevent re-occurrence of the arrhythmia.

In one embodiment, subcutaneous electrical stimulation may be initiated prior to performing exercise, assuming a patient has an implantable medical device implanted within his body. Such stimulation appears to result in a short-term inhibition of the sympathetic outflow of the heart, which in turn causes changes in the neural chemistry in a manner that prevents damage from ischemic conditions. Stimulation may be provided for a predetermined length of time, which in one embodiment is approximately thirty minutes, shortly prior to performing the cardiac procedure or engaging in exercise. The amount of stimulation may also be selected based on the anticipated level of exertion.

In another embodiment, subcutaneous electrical stimulation may be performed at upper cervical levels C1-C3 instead of at T1-T4. Although stimulation of this area has typically been performed to reduce jaw and neck pain or occipital neurolgia, it has been found such stimulation, can also reduce angina, and can provide important cardiac protection when performed prior to a cardiac insult. In yet another embodiment, stimulation may be performed at C2 and C3 instead.

In another embodiment, stimulation may be automatically initiated because of physiological measurements obtained by the controller 104. That is, controller 104 may utilize one or more conventional sensors such as sensor s 110 and 111 to sense signals that predict the possible on-set of physiologic conditions such as ventricular dysfunction, ischemia, heart failure, or any other type of cardiac insult. These sensors may be any of the types known in the art for sensing physiological signals, including pressure, oxygen, activity, temperature, and blood flow sensors. Exemplary sensors are disclosed in U.S. Pat. No. 4,903,701 issued to Moore et al., U.S. Pat. No. 5,564,434, issued to Flalperin et al, U.S. Pat No. 4,428,378, issued to Anderson et al., U.S. Pat. No. 5,464,434, issued to Alt or U.S. Pat. No. 5,330,505, issued to Cohen.

Upon anticipation or detection of the cardiac event, the controller 104 may automatically begin therapeutic treatment of the patient by subcutaneous electrically stimulating the selected nervous tissue(s).

In the embodiment wherein controller 104 is an external device, any type of external physiological sensor system known in the art may be utilized within the scope of the current invention. This may include, for example, externally-placed electrodes for measuring ECG signals in a manner known in the art. Other examples include pressure and temperature sensors, and/or sensors that may externally measure blood chemistry.

After treatment is initiated, therapy may continue during an insult. Such stimulation could be continued until a cardiovascular intervention procedure is initiated, or even continued for several weeks past the incident.

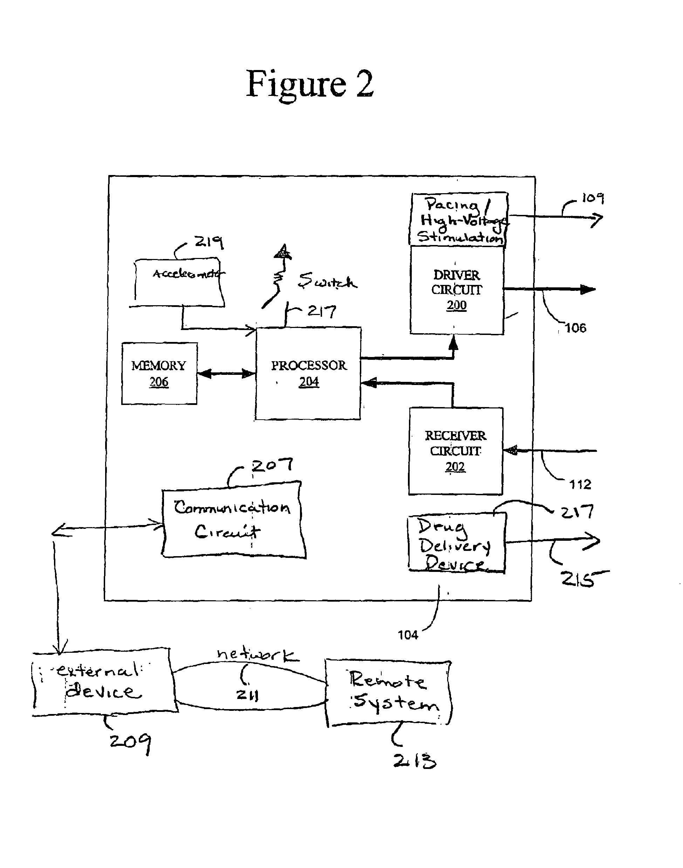

Figure 2 illustrates a block diagram of one embodiment of the controller 104. Generally, the controller 104 is comprised of one or more driver circuits 200 and receiver circuits 202. The driver circuits 200 are generally responsible for providing the stimulating signals over the lines 106 to the electrodes 108. That is, a processor 204, operating under software or hardware control, may instruct the driver circuit 200 to produce a stimulating signal having a set of preselected, desired parameters, such as frequency, duty cycle, duration, waveform shape, amplitude, voltage and magnitude. As noted above, driver circuits 200 may optionally include circuits 201 to generate pacing and/or high-voltage stimulation to the heart on leads 109.

The receiver circuits 202 are generally responsible for receiving signals from the sensors 110 and 111, and processing those signals into a form, such as a digital format, which may be analyzed by the processor 204 and/or stored in a memory 206, such as a dynamic random access memory (DRAM). The memory 206 may also store software, which is used to control the operation of the processor 204.

In one embodiment wherein controller 104 is included in an implanted device, signals stored in memory 206 may be transferred via a communication circuit 207 such as a telemetry circuit to an external device 209 such as a programmer. These signals may be stored in the external device, or transferred via a network 211 to a remote system 213 which may be a repository or some other remote database. Network 211 may be an intranet, internet system such as the world-wide web, or any other type of communication link.

Controller 104 may further include a reed switch 217. This type of switch mechanism may be closed using a magnet in the embodiment wherein the controller is implanted within a patient's body. Alternatively, another type of patient-activated mechanism such as an accelerometer 219 may be utilized for detecting a tapping sequence to activate the implantable embodiment of the invention. This type of tapping mechanism is known in the art.

As noted above, controller 104 may further include a drug delivery device 213 that may comprise a pump coupled to a catheter 215. Exemplary implantable drug delivery systems that may be adapted to deliver biologically-active agents in conjunction with delivery of the subcutaneous stimulation are disclosed in U.S. Pat. No. 5,607,418, issued to Arzbaecher, U.S. Pat. No. 5,220,917, issued to Cammilli, U.S. Pat. No. 4,146,029, issued to Ellinwood and U.S. Pat. No. 5,330,505, issued to Cohen.

As noted above, in one embodiment, delivery of the subcutaneous stimulation may be modified based on a variety of measurable physiologic parameters used in a closed loop control system. As depicted in Figures 1A, 1B, and 1C representative sensor 110 or 111 may be positioned adjacent or within the body of the patient 102 to sense various physiological conditions, which are communicated back to the controller 104. The measured physiological conditions may be used as an indication of the patient's response to the therapy being administered by the controller 104. That is, a positive physiological response may be used as an indication that the therapy is achieving the desired result. The sensed physiological conditions may be used to adjust the parameters of the stimulation. For example, the controller 104 may measure and record cardiac pulse pressure. A change in the cardiac pulse pressure over time may be used in a closed-loop system to adjust delivery of stimulation. For example, if the controller 104 detects that the cardiac pulse pressure has declined over time, then the parameters of the stimulation may be adjusted in an attempt to increase the cardiac pulse pressure. On the other hand, where the controller 104 observes a consistent, appropriate cardiac pulse pressure, then the stimulation may be continued, as a desired result is being achieved by the stimulation. On the other hand, where the controller 104 observes continued high, or even rising, cardiac pulse pressure, then the parameters of the stimulation may be adjusted in an attempt to lower the cardiac pulse pressure over time.

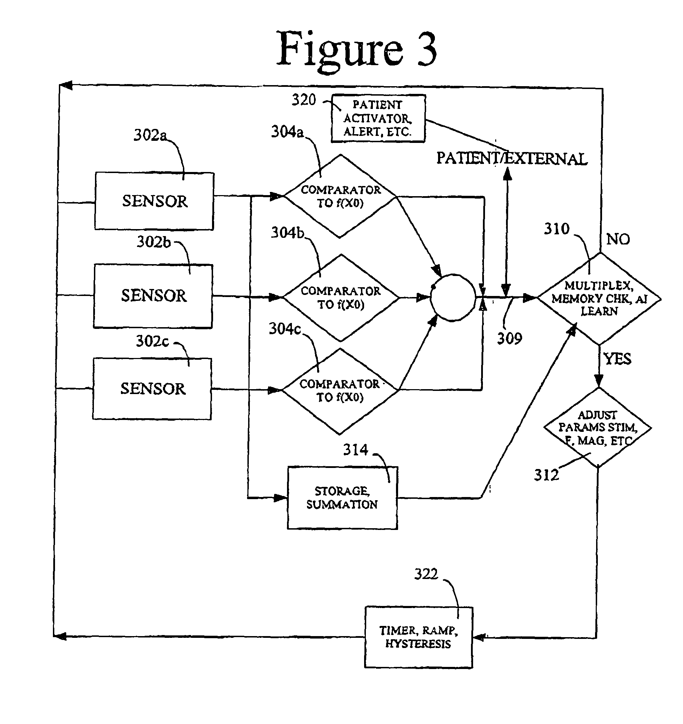

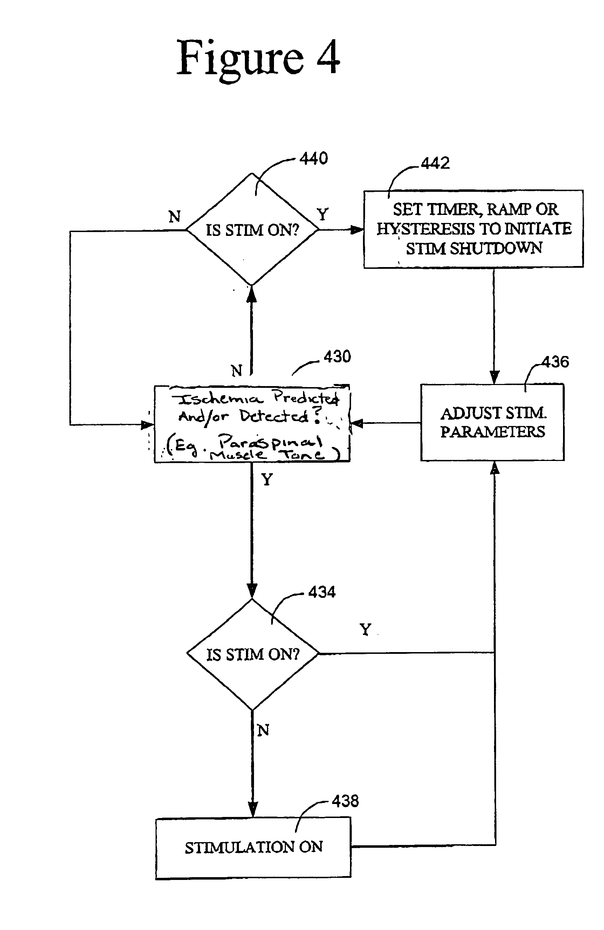

The overall general operation of the controller 104 may be appreciated by reference to a control diagram and flowchart depicted in Figures 3 and 4. Those skilled in the art will appreciate that the control diagram and flowchart illustrated herein may be used to represent either software that may be executed by the processor 204 or hardware configured to operate to perform the functions set forth in the flowchart. Thus, either hardware or software may be employed without departing from the scope of the instant invention.

Figure 3 depicts a generalized mode of closed loop operation. Through a sensor or combination of sensors, the system evaluates a physiologic state. This includes predicting (and later, detecting the continuation of) ischemia, an increased risk of VT/VF, a cardiovascular decompensation, and/or other types of cardiac insults to be discussed below. Any of the sensing systems listed below may be used to monitor physiological parameters to accomplish this function.

In response to the detection of a particular physiologic state, the system adjusts the stimulation parameters to treat the detected or predicted abnormality. The system may also record trends in the sensed data and the effects or impact of a prior stimulation intervention. In one embodiment, the system may include an artificial intelligence system that allows the device to learn from the effectiveness of the prior therapy. The system thereby becomes customized to deliver therapy that is optimally tailored for the individual patient.

After stimulation is initiated in response to an anticipated or detected insult, stimulation parameters may be adjusted. Such parameters may include stimulation pulse width, amplitude, frequency, duty cycle, and waveform shape. These parameters may be continually modified as the response is monitored so that the optimal treatment may be delivered. After the insult such as an ischemic episode has subsided, stimulation may be discontinued after an appropriate delay. A ramp-down process may be provided to allow for some hysteresis. Sensed data and device parameters may be transferred to an external device such as a programmer using a communication system such as a telemetry circuit. The physician may then evaluate the data and determine whether the delivered therapy requires modification, and whether it is desirable to enable the device to provide patient-initiated therapy in a manner to be discussed below. Additionally, the data may provide valuable information that may be used to deliver more effective manual therapy.

In Figure 3, one or more sensors shown as sensors 302a through 302c are used to measure physiologic conditions. The measured signals may be compared against a threshold value by one or more comparators 304a through 304c. The results of the comparisons may be summed, or otherwise processed, with the processed data set being provided on line 309. If this result indicates that electrical stimulation is required, as determined by block 310, therapy is initiated. Therapy is initiated and controlled by a processing circuit, as represented by block 312. This processing circuit 312 provides the closed-loop feedback control used to modulate the level of therapy delivered. When therapy is to be discontinued, a ramp-down circuit shown in block 322 may be used to gradually discontinue the stimulation.

In one embodiment, artificial intelligence capability may be provided by the logic of block 310. This artificial intelligence analyzes the effectiveness of previously delivered therapy to adjust current therapy delivery techniques. Therapy is thereby tailored to individual patient needs.

According to another manner of initiating therapy, the signals provided by the sensors 302a through 302c may be combined to generate a cumulative signal indicative of a patient's overall physiologic condition. This may be accomplished using a summation circuit 314, for example. The cumulative signal may be provided along with, or in place of, the signal on the line 309 for use in determining whether therapy should be initiated or modulated. In addition to closed-loop operation, Figure 3 also includes open-loop methods of initiating therapy, including patient-initiated therapy shown in block 320.

Figure 4 illustrates a flowchart representation of one embodiment of operating a closed-loop system according to the current invention. In block 430 of Figure 4, a determination is made as to whether ischemia is anticipated. This determination is based on monitored physiological parameters that may include detection of physical activity, a change in the ST segment, change in paraspinal muscle tone, and/or a change in heart rate. Other parameters may be monitored in a manner to be discussed further below.

According to one aspect of the invention, electrical stimulation is provided when the tone in the paraspinal muscles is increasing, since this is an indicator of anticipated visceral complications. Detection of this increase in muscle tone could be accomplished using an externally-positioned strain gage, for example. Thus, electrical stimulation may be applied prior to the onset of actual ischemic so that cardiac tissue maybe protected in an anticipatory manner. Electrical stimulation may also continue while the muscle tone remains at a predetermined rigidity. In one embodiment, a rate-responsive sensor such as an accelerometer or other appropriate sensor may be used to sense the level of activity, and adjust the stimulation levels according to the activity level.

If ischemia is anticipated, and the stimulation has already been initiated as detected by block 434, the stimulation level may be adjusted in block 436 based on the monitored parameters. This may include adjusting the rate, amplitude, duration, or waveform shape of electrical stimulation pulses applied to the electrodes 108. If stimulation has not yet been initiated, it may be activated in block 438. If artificial intelligence is provided, the level and/or type of stimulation may be correlated with the physiologic result of the stimulation so that therapy may be adjusted in the future. The stimulation may be modulated in block 436, with the monitoring of patient condition continuing in block 430.
Stimulation may continue after the ischemia is actually detected.

If ischemia is not anticipated and/or detected in block 430, and stimulation is activated, as indicated by block 440, stimulation may be discontinued, as shown in block 442. In one embodiment this may be accomplished using a timer and a ramp-down mechanism to gradually disable the stimulation therapy.

As noted above, a closed-loop system may be utilized to control initiation and delivery of the subcutaneous electrical stimulation. The closed-loop system may utilize one or more physiological sensors known in the art to sense one or more physiological conditions that will be utilized to control therapy. Such sensors may include activity sensors, sensors for detecting cardiac electrical or mechanical activity, mechanisms for detecting autonomic activity or hemodynamic parameters, sensors for measuring blood chemistry, and mechanisms for tracking time-of-day. A partial exemplary listing of select types of sensing mechanisms that may be utilized in the closed-loop system for predicting cardiac insults are summarized in Table 1 below. The following table summarizes the types of sensors that may be employed to predict and/or detect a corresponding physiologic condition. Any one or more of the sensing devices and/or other sensing mechanisms known now or in the future for sensing physiological parameters may be employed without departing from the scope of the current invention.

In Table 1, column 1 lists general categories of sensors, column 2 corresponds to a particular physiologic parameter that may be monitored, column 3 outlines a corresponding sensor used to monitor the parameter, and column 4 relates to the type of physiologic condition or occurrence that may be anticipated using the measurement.

**Table I. Physiological Parameters to be Sensed or Monitored**

| GENERAL MODALITY | SPECIFIC ITEMS | SENSING METHODS | WHAT IT CORRESPONDS TO |
|---|---|---|---|
| Physical Activity | Posture | Gravity direction, accelerometer | Posture |
| | Ambulation/Motion Detector | Piezoelectric Crystal, accelerometer | Motion |
| | Minute Ventilation | Impedance | Respiration (rate and volume) |
| | Temperature | Thermistor | Body temperature |
| | Blood changes with activity | PO2, SA02, pH, Catecholamines, adrenalin | Blood chemistry |
| Cardiac Electrical Activity | Changes in Morphology of Complexes (QRS, T waves) | ECG, Intracardiac Electrogram (EGM), subcutaneous Electrogram (EGM) | Changes in cardiac depolarization or repolarization patterns |
| | Repolarization Alternans, T Wave Alternans, QRS Alternans, ST Segment Alternans | ECG, Intracardiac EGM subcutaneous EGM | Abnormalities on cardiac electrical depolarization, and repolarization |
| | Heart rate & rhythm (NSVT episodes of VT/VF, PVC's heart rate variability) | ECG, Intracardiac EGM subcutaneous EGM | Cardiac rhythms, regularity |
| | Changes in AV Interval, AV Interval variability, dynamic responses of AV interval to changes in HR Changes in QT Interval QT Interval variability, Responses of QT Interval to changes in HR | ECG, Intracardiac EGM subcutaneous EGM | Cardiac conduction abnormalities, autonomic and paracrine modulation of same |
| | | ECG, Intracardiac EGM subcutaneous EGM | Cardiac repolarization autonomic and paracrine modulations of same |
| Cardiac ischemia | ST Segnment changes, Q Wave, QRS magnitude And width, | ECG, Intracardiac EGM subcutaneous EGM, blood chemistry (see below) | Mycardial perfusion (balance between supply and demand) |

| GENERAL MODALITY | SPECIFIC ITEMS | SENSING METHODS | WHAT IT CORRESPON DSTO |
|---|---|---|---|
| Neutral Activity | EEG | Cortical motor strip | Global neutral activity |
| | EMG | Paraspinal muscles | Increases indicate cardiac stress |
| | | Other muscles | |
| | Heart rate turbulence | | Sinus arrhythmia |
| Hemodynamic Parameters | Arterial or Venous Pressure | Pressure transducer | Systolic Diastolic and Pulse pressure; central venous pressure |
| | Cardiac chamber pressures | Pressure transducer | Developed pressures, peak systolic, diastolic pressures, dP/dt |
| | Cardiac mechanical activity | Accelerometer, sonomicrometer crystals | Tissue displacement, coordination, contraction |
| Blood Chemistry (central arterial and local tissue and differences between these) | PO₂, SAO₂ | Oximetry, O₂ Probe | Related to cardiac performance |
| | Gluecose | Oximetry | Indicator of Myocardial Metabolism |
| | Lactate | Oximetry | Indicators of Myocardial Metabolism |
| | PC O₂ | C O₂Probe | Related to cardiac performance |
| | pH | pH Probe | Abnormalities may indicate myocardial electrical instability |
| | Troponin | Molecular Probe | Indicators of Myocardial Ischemia |
| | CKMB | Molecular Probe | Indicators of Myocardial Ischemia |
| | Electrolytes | Molecular Probe | Abnormalities may indicate myocardial electrical instability |
| | Drug levels | Molecular Probe | As indicators of level of protection provided by drug (e.g. antiarrhythmic s) |
| | Catecholamines | Molecular Probe | Autonomic Activity/Tone |
| | NO or precursors | Molecular Probe | Related to cardiac injury |
| | Endogenous opiates | Molecular Probe | Autonomic Activity/Tone |
| Time of Day | Clock/Date | Track because activity and risk vary during day or year | |

In one embodiment, electrical stimulation is provided to peripheral nerves in dermatones T1- T12, C1 -C8, or other areas of the spinal cord. Any combination of these sites may be stimulated. Such stimulation may involve electrodes implanted outside the vertebral canal at the desired location. In another embodiment, the vagus and/or peripheral nerve may be stimulated at various locations. If desired, stimulation may be provided subcutaneously located in the precordial area or over sites of the pain or any area from which nervous fibers project to the spinal cord at levels T1-T5.

The sites of stimulation may include the following, with any combination being utilized:
a. Nerves near the vertebral canal (T1 - T12, preferably T1-T4; C 1-C8);
b. Vagus Nerve;
c. Chest wall (precordial, near median nerve, toward muscle);
d. Peripheral Nerve (median, peritoneal, ulnar, C2 and C3, ansa lenticularis, dorsal root ganglia);
e. Carotid sinus, and other cranial nerves;
f. Sympathetic ganglia; and
g. Intrinsic cardiac neurons.

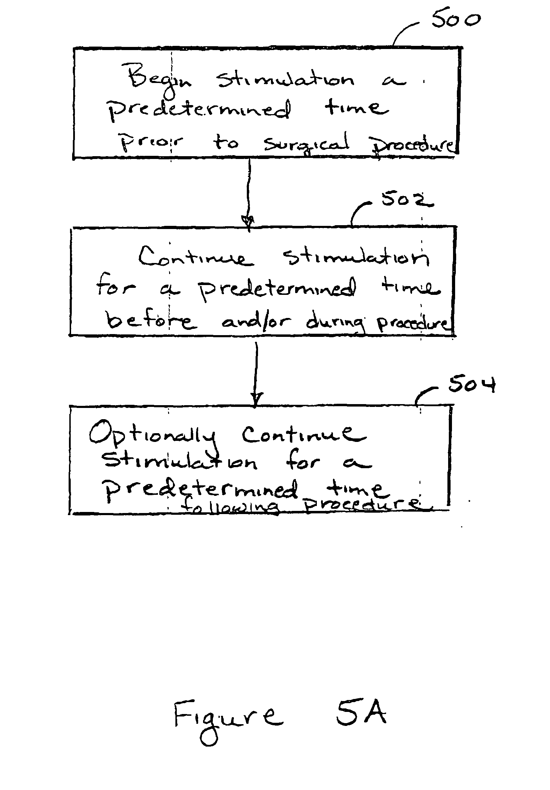

Electrical stimulation provides significant benefits when delivered prior to an anticipated cardiac insult, or an event that will induce ischemia. The benefits include minimizing or preventing acute infarct and reducing reperfusion arrhythmia. In one embodiment, the therapy is delivered thirty minutes or more prior to the anticipated on-set of an insult such as ischemia. As much as possible, the above therapies should be implemented prior to the insult. Some of the many exemplary embodiments included within the scope of the invention are shown in Figures 5A through 5E.

Figure 5A is a flowchart illustrating delivery of subcutaneous stimulation prior to planned cardiac interventions, like bypasses, angioplasties or stents (block 500). The stimulation could be applied for a predetermined time such as 30 - 120 minutes prior to the intervention (block 502). Stimulation may be continued for hours or days after the procedure to minimize adverse effects or to increase or even maximize patency of vessels (block 504).

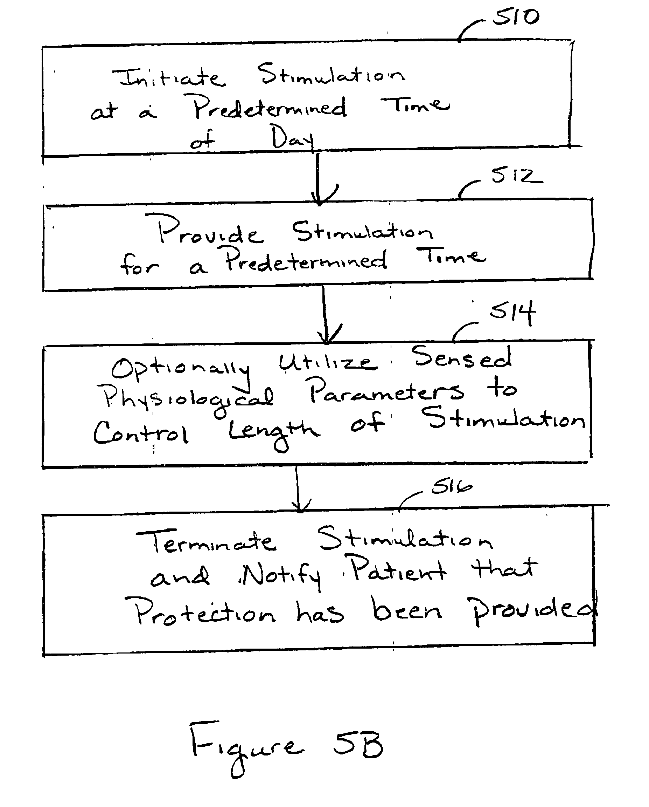

Figure 5B is a flowchart illustrating delivery of stimulation at a particular time of day (block 510). For example, stimulation may be provided when a patient wakes up in the morning. A timer may be utilized to initiate subthreshold stimulation, or alternatively, to initiate suprathreshold stimulation to provide paresthesia. After a predetermined time such as thirty minutes (block 512), or when sensed physiological parameters indicate that the appropriate level of cardiovascular protection has been established (block 514), the patient can be alerted (516). This could be accomplished, for example, by use of stimulation producing a stronger paresthesia.

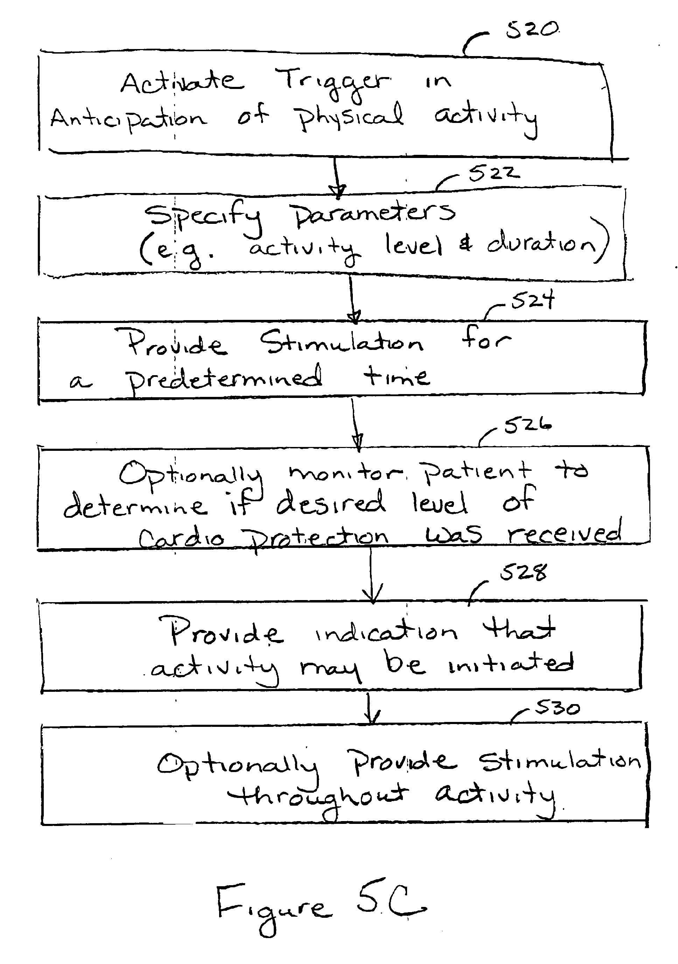

Figure 5C is a flowchart illustrating delivery of stimulation initiated because a patient anticipates physical activity and manually triggers therapy (block 520). This may be accomplished using an externally-positioned magnet as may be used to close a reed switch. Alternatively, a tapping sequence may be initiated as is known in the art. In this embodiment, the patient performs a tapping action over the implanted device as may be accomplished using a finger. This tapping action is detected by an accelerometer or similar sensor within the device so that therapy may be initiated.

In one embodiment, an expected intensity of the activity or other optional parameters may also be specified (block 522). After stimulation has been delivery for the specified time (block 524) and/or after the appropriate level of cardio protection has been determined to have been established (block 526), the device provides an indication that activity may be initiated (block 528). Stimulation may continue throughout the activity, if desired (block 530).

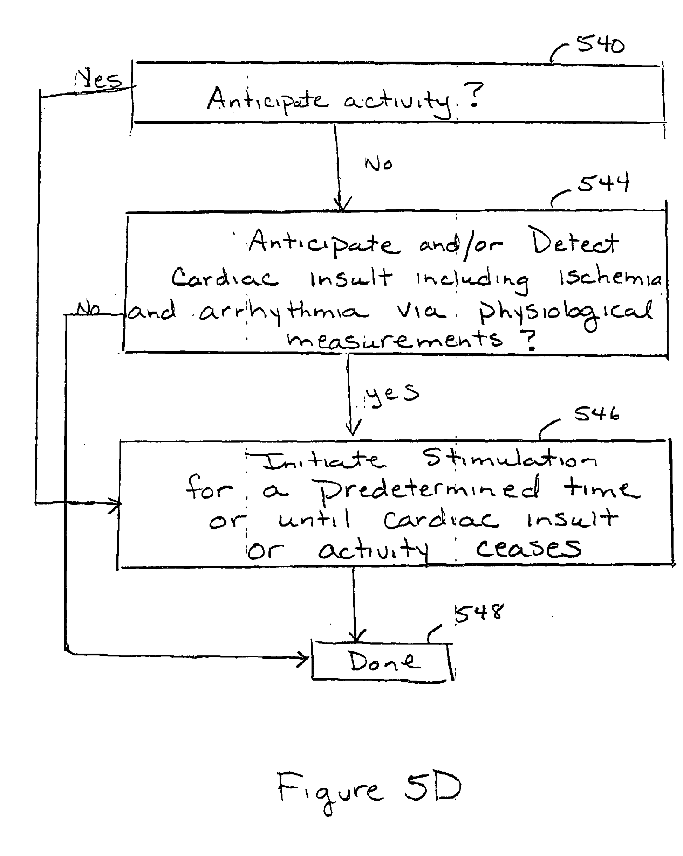

Figure 5D is a flowchart illustrating stimulation initiated at the first signs of activity in an anticipatory manner (block 540), or at the first indication that ischemia, an episode of malignant ventricular arrhythmia, and/or any of the other insults discussed above may be anticipated (block 544). This type of indication may be detected by one or more of the sensing mechanisms discussed above.

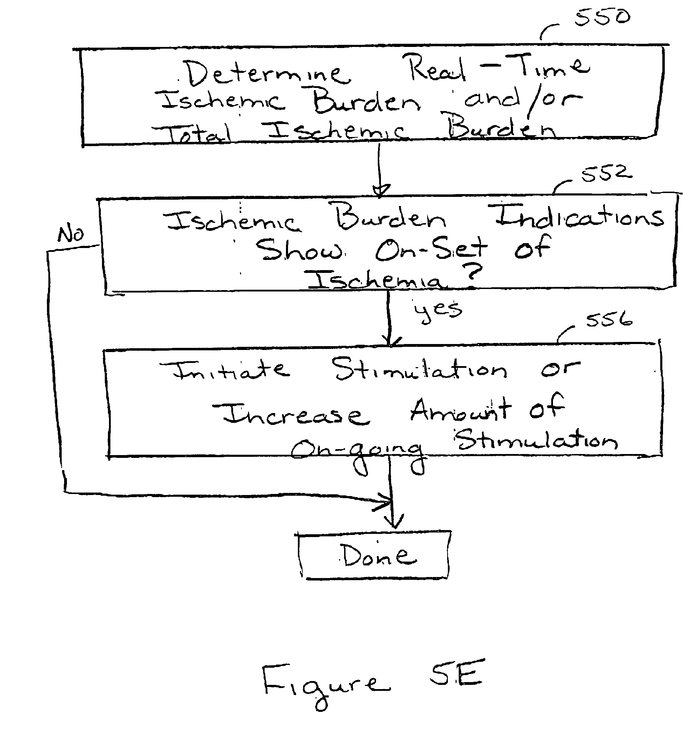

Figure 5E is a flowchart illustrating stimulation initiated based on a real time recording of ischemic burden and total ischemic burden (blocks 550 and 552). If desired, the prophylactic amount of stimulation could be increased if these measurements show increased ischemia in general, or an increased likelihood of the onset of ischemia (block 556).

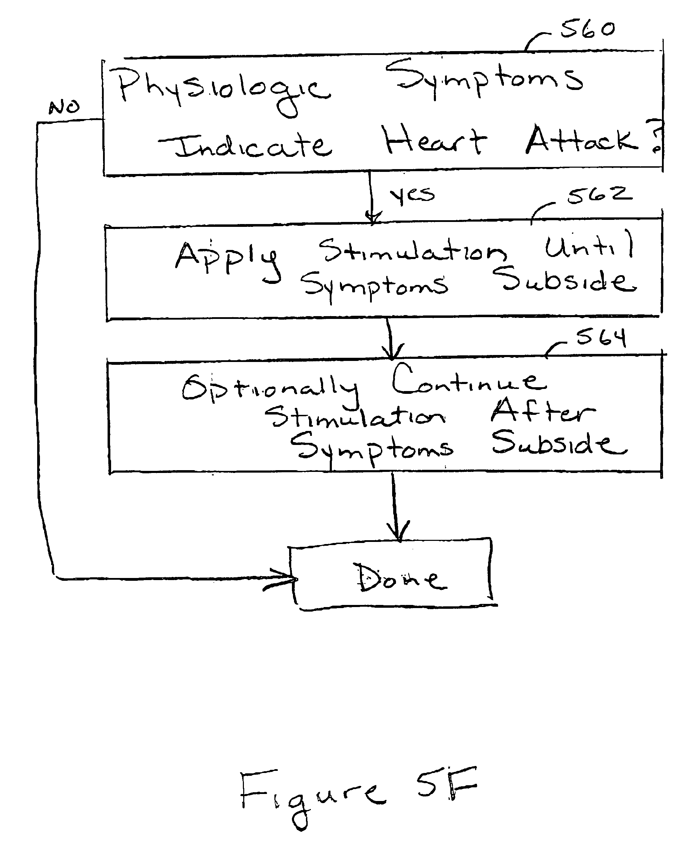

Figure 5F illustrates the delivery of the therapy for protection during a suspected heart attack. To promote optimal recovery, stimulation may be applied by healthcare professionals as soon as possible in an appropriate form if a heart attack is even suspected (blocks 560 and 562). This is done using transcutaneously-inserted subcutaneous electrode systems discussed above. This stimulation may continue after the symptoms subside to further protect the cardiac tissue (564).

Table II illustrates some of the benefits associated with the subcutaneous electrical stimulation provided by the current invention. Table II further lists one or more physiological parameters that may be monitored when delivering stimulation to achieve a desired effect.

**Table II - Benefits of Stimulation**

| BENEFITS | PHYSICOLOGICAL PARAMETERS TRACKED |
|---|---|
| Prevention of VT / VF Incidents | Cardiac electrical, Cardiac Ishemia, Autonomic Activity, Physical Activity, Heart Rate and Rhythm |
| Reduce PVC's | Cardiac electrical, Cardiac Ishemia, Autonomic Activity, Physical Activity, Heart Rate and Rhythm |
| Reduce NSVT | Cardiac electrical, Cardiac Ishemia, Autonomic Activity, Physical Activity, Heart Rate and Rhythm |
| Lessen Cardiac Ischemia | Cardiac Ischemia; total ischemic burden, Physical Activity |
| Reduce Angina | Physical Activity, Cardiac Ishemia |
| Improved Exercise Tolerance | Physical Activity, respiration, blood chemistry |
| Rebalance Autonomic System | Cardiac electrical, Autonomic Activity, Hemodynamics |
| Improve Cardiac Performance: pump function, preload/afterload | Cardiac electrical and hemodynamics |
| Improve Cardiac Paracrine Function or Balance | Cardiac electrical and hemodynamics |
| Alter AV electrical function | Cardiac electrical |
| Restore heart rate Variability | Cardiac electrical, Autonomic Activity |
| | |

The above-described closed-loop system may combine subcutaneous electrical stimulation with conventional drug therapy. The drug therapy may be provided by an implanted delivery device such as that discussed above, for example. The closed-loop system may be utilized to titrate the drug delivery and the stimulation in much the same manner as discussed above in conjunction with the closed loop electrical stimulation.

As discussed in detail above, one aspect of the inventive system provides a system for employing closed-loop controls to initiate and deliver subcutaneous electrical stimulation. However, as also indicated above, the invention may also be utilized in an open-loop mode wherein the stimulation is triggered by the patient or another person. As shown in Figure 3, the system may also provide the ability for the patient to activate the stimulation based on the onset of a physical condition such as exertion or pain. Patient-initiated therapy may be limited or controlled by a programmable feature as specified by a physician. A timer may also be provided to initiate and control therapy at one or more times during the day.

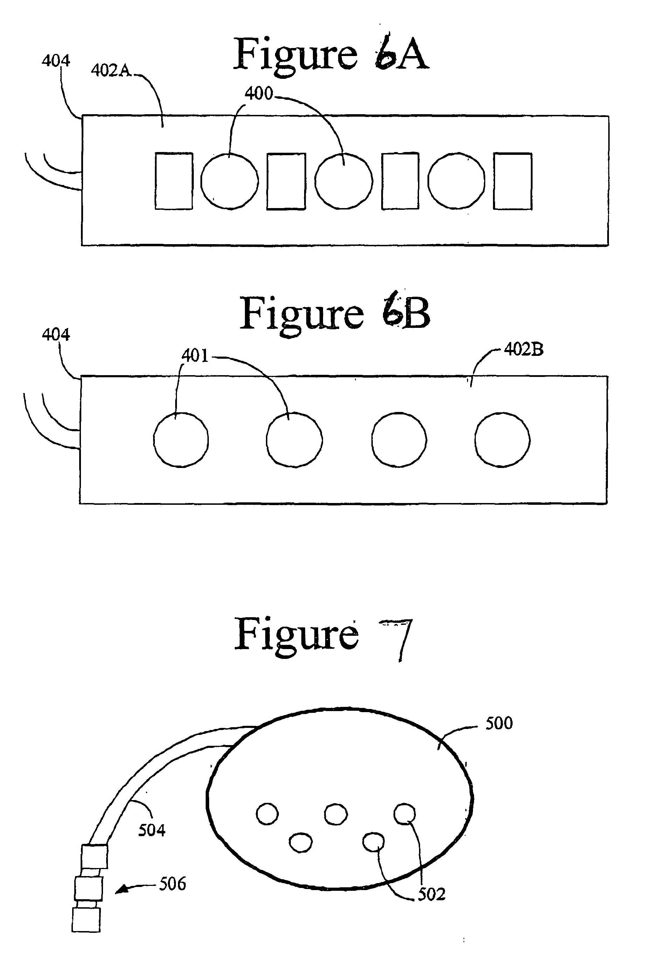

Any type of subcutaneous electrode system know in the art may be utilized in an apparatus not according to the current invention. In one embodiment of the invention, as shown in Figures 6A and 6B, an electrode 400, 401 may be provided on a first and second side 402A, 402B of a lead 404, with the electrodes 400, 401 employed for stimulation at a given time being selectively enabled by a user. For example, the lead 404 may be positioned subcutaneously with the first side 402A positioned to face skin and the second side 402B positioned facing the underlying muscle. The electrodes 400, 401 may then be selectively energized to stimulate nerve tissue in the skin, muscle, or both. Alternatively, the system may be programmable to select the type of tissue to be stimulated. This is desirable since in some instances, it may be beneficial to provide stimulation to only spinal neurons, whereas in other instances it may be desirable to also stimulate skin, muscle, or any combination of the nervous tissues. Various electrode combinations could be provided to allow for selective enabling of the stimulation in this manner.

In another embodiment, the paddle-type lead may be between four and ten millimeters wide so as to be readily passable through a twelve-gage needle before it unfolds. That is, a special needle may be provided having an oval or rectangular cross-section of appropriate size to allow for the delivery of this type of lead. Electrodes are provided on both sides of the paddle lead.

In the case of an implantable device, as shown in Figure 7, a housing 500 may have a plurality of electrodes 502 formed thereon or attached thereto. The housing 500 may be positioned during implantation to excite skin, muscle, or both. In the illustrated apparatus not according to the invention the implantable device also includes an optional accessory lead 504 with a plurality of electrodes 506 positioned thereon. The electrodes 506 may be inserted into a subcutaneous space to provide additional or sole excitation of nervous tissue located remote from the implantable device.

Subcutaneous electrodes of the type shown in Figure 7 may take several forms.
For example, commonly assigned U.S. Patent No. 5,292,338.
describes a system that may include an electrode included within a surface of the defibrillator housing. An alternative design is described in commonly-assigned U.S. Patent No. 5,331,966. The alternative design utilizes electrodes insulated from the housing. These electrodes may be adapted to provide stimulation to subcutaneous tissue in the manner discussed above.

In one embodiment, a notification feature is provided to notify the patient and/or a physician of changing patient conditions indicative of increased ischemic risk. The invention may further include means to discontinue or limit therapy when closed-loop feedback techniques are leading to an undesirable situation.

## Claims

1. An apparatus for protecting cardiac tissue from insult, comprising:
a paddle lead (404) coupled to first and second subcutaneous electrodes (400, 401), said first electrode (400) being provided on a first side (402A) of said lead and said second electrode (401) being provided on a second side of said lead, said electrodes being positionable proximate to nerve tissue; and
a controller (104) adapted to deliver electrical stimulation to the first and second electrodes.

2. The apparatus of claim 1 for protecting cardiac tissue from insult, wherein:
said first and second electrodes (108) are positionable proximate to one or more of the group consisting of peripheral nerves, intrinsic cardiac nerves, sympathetic ganglia, and cranial nerves.

3. The apparatus of claim 1 or 2, and further, wherein said first and second electrodes include a surface to provide stimulation to at least one of a region of skin, muscle tissue, and spinal neurons of the patient's body.

4. The apparatus of claim 1, 2 or 3, further comprising memory (206) adapted to store data descriptive of the electrical stimulation, and wherein the controller is adapted to analyze the stored data and adjust electrical stimulation in response thereto.

5. The apparatus of any preceding claim, further comprising a sensor (111) configured to detect a physiologic condition representative of an operating characteristic of the patient's heart, and wherein the controller is adapted to deliver electrical stimulation to the electrodes based on an indication of the physiologic condition.

6. The apparatus of any preceding claim, wherein the controller includes a circuit (204) to control delivery of electrical stimulation to the electrodes for a preselected duration of time.

7. The apparatus of claim 1 for protecting cardiac tissue from insult, comprising:
wherein said electrodes are capable of stimulating at least a portion of a nervous system of the body;
trigger means (217, 219); and
means for delivering electrical stimulation to the electrodes in response to activation of the trigger means.

8. The apparatus of claim 7, wherein the trigger means includes means for being activated by a person.

9. The apparatus of claim 7 or 8, and further including a sensor (110, 111) to measure a physiological signal, and wherein the trigger means includes means for being activated based on a predetermined condition indicated by the physiological signal.

10. The apparatus of claim 9, wherein the means for delivering electrical stimulation including means for delivering the electrical stimulation for a predetermined period of time.

11. The apparatus of claim 7, 8, 9 or 10 wherein the trigger means includes means for being activated in anticipation of the insult.

## Patentansprüche

1. Vorrichtung zum Schutz von Herzgewebe vor Insult mit:
einer Paddelleitung (404), die an erste und zweite subkutane Elektroden (400, 401) angeschlossen ist, wobei die ersten Elektrode (400) auf einer ersten Seite (402A) der Leitung vorgesehen ist und die zweite Elektrode (401) auf einer zweiten Seite der Leitung vorgesehen ist, wobei die Elektroden in der Nähe von Nervengewebe positionierbar sind; und
einem Steuergerät (104), das dafür eingerichtet ist, elektrische Stimulation an die ersten und zweiten Elektroden abzugeben.

2. Vorrichtung nach Anspruch 1 zum Schutz von Herzgewebe vor Insult, wobei:
die ersten und zweiten Elektroden (108) in der Nähe von einem oder von mehreren Elementen aus der Gruppe anordenbar sind, die aus peripheren Nerven, intrinsischen Herznerven, sympathischen Ganglien und Cranialnerven besteht.

3. Vorrichtung nach Anspruch 1 oder 2, wobei die ersten und zweiten Elektroden ferner eine Oberfläche zur Bereitstellung von Stimulation an wenigstens eine Hautregion, Muskelgewebe und/oder Spinalneuronen des Körpers des Patienten aufweisen.

4. Vorrichtung nach Anspruch 1, 2 oder 3, die ferner einen Speicher (206) aufweist, der dafür eingerichtet ist, Daten zu speichern, die bezüglich der elektrischen Stimulation beschreibend sind, und wobei das Steuergerät dafür eingerichtet ist, die gespeicherten Daten zu analysieren und elektrische Stimulation als Antwort hierauf einzustellen.

5. Vorrichtung nach einem der vorstehenden Ansprüche, die ferner einen Sensor (111) aufweist, der dafür eingerichtet ist, einen physiologischen Zustand zu bestimmen, der bezüglich eines Funktionscharakteristikums des Herzens des Patienten kennzeichnend bzw. repräsentativ ist, und wobei das Steuergerät dafür eingerichtet ist, elektrische Stimulation an die Elektroden auf Grundlage einer Anzeige des physiologischen Zustands abzugeben.

6. Vorrichtung nach einem der vorstehenden Ansprüche, bei der das Steuergerät eine Schaltung (204) zum Steuern der Abgabe elektrischer Stimulation an die Elektroden für eine ausgewählte Zeitdauer beinhaltet.

7. Vorrichtung nach Anspruch 1, zum Schutz von Herzgewebe vor Insult, die beinhaltet:
wobei die Elektroden dazu in der Lage sind, wenigstens einen Teil eines Nervensystems des Körpers zu stimulieren;
Auslösermittel (217, 219); und
Mittel zur Abgabe elektrischer Stimulation an die Elektroden als Antwort auf die Aktivierung der Auslösemittel.

8. Vorrichtung nach Anspruch 7, bei der die Auslösermittel Mittel zum Aktiviertwerden bzw. zur Aktivierung durch eine Person beinhalten.

9. Vorrichtung nach Anspruch 7 oder 8, die ferner einen Sensor (110, 111) zur Messung eines physiologischen Signals beinhaltet und wobei die Auslösermittel Mittel zur Aktivierung auf der Grundlage eines vorbestimmten Zustands, der durch das physiologische Signal angezeigt wird, beinhalten.

10. Vorrichtung nach Anspruch 9, bei der die Mittel zur Abgabe elektrischer Stimulation Mittel zur Abgabe elektrischer Stimulation für einen vorbestimmten Zeitabschnitt beinhalten.

11. Vorrichtung nach Anspruch 7, 8, 9 oder 10, bei der die Auslösermittel Mittel zur Aktivierung unter Vorwegnahme bzw. in Antizipation des Insults beinhalten.

## Revendications

1. Dispositif destiné à protéger un tissu cardiaque contre une atteinte ou lésion, comportant :
un fil de type palette (404) couplé à des première et seconde électrodes sous-cutanées (400, 401), ladite première électrode (400) étant agencée sur un premier côté (402A) dudit fil, et ladite seconde électrode (401) étant agencée sur un second côté dudit fil, lesdites électrodes pouvant être positionnées à proximité d'un tissu nerveux ; et
un contrôleur (104) adapté pour délivrer une stimulation électrique aux première et seconde électrodes.

2. Dispositif selon la revendication 1 destiné à protéger un tissu cardiaque contre une lésion, dans lequel :
lesdites première et seconde électrodes (108) peuvent être positionnées à proximité d'un ou plusieurs éléments parmi le groupe constitué de nerfs périphériques, de nerfs cardiaques intrinsèques, de ganglions sympathiques et de nerfs crâniens.

3. Dispositif selon la revendication 1 ou 2, et également, dans lequel lesdites première et seconde électrodes incluent une surface pour assurer la stimulation d'au moins un élément parmi une région de la peau, un tissu musculaire et des neurones spinaux du corps du patient.

4. Dispositif selon la revendication 1, 2 ou 3, comportant en outre une mémoire (206) adaptée pour mémoriser des données descriptives de la stimulation électrique, et dans lequel le contrôleur est adapté pour analyser les données mémorisées et ajuster la stimulation électrique en réponse à celles-ci.

5. Dispositif selon l'une quelconque des revendications précédentes, comportant également un capteur (111) configuré pour détecter une condition physiologique représentative d'une caractéristique de fonctionnement du coeur du patient, et dans lequel le contrôleur est adapté pour délivrer une stimulation électrique aux électrodes sur la base d'une indication de la condition physiologique.

6. Dispositif selon l'une quelconque des revendications précédentes, dans lequel le contrôleur inclut un circuit (204) pour commander une délivrance de stimulation électrique aux électrodes pendant une période de temps présélectionnée.

7. Dispositif selon la revendication 1 destiné à protéger un tissu cardiaque contre une atteinte ou lésion, comportant :
lesdites électrodes étant capables de stimuler au moins une partie d'un système nerveux du corps ;
des moyens de déclenchement (217, 219) ; et
des moyens pour délivrer une stimulation électrique aux électrodes en réponse à une activation des moyens de déclenchement.

8. Dispositif selon la revendication 7, dans lequel les moyens de déclenchement incluent des moyens destinés à être activés par une personne.

9. Dispositif selon la revendication 7 ou 8, et incluant également un capteur (110, 111) pour mesurer un signal physiologique, et dans lequel les moyens de déclenchement incluent des moyens destinés à être activés sur la base d'une condition prédéterminée indiquée par le signal physiologique.

10. Dispositif selon la revendication 9, dans lequel les moyens pour délivrer une stimulation électrique incluent des moyens pour délivrer la stimulation électrique pendant une période de temps prédéterminée.

11. Dispositif selon la revendication 7, 8, 9 ou 10 dans lequel les moyens de déclenchement incluent des moyens destinés à être activés en anticipation de la lésion.
